# EUROPEAN PATENT SPECIFICATION

(11) **EP 0 240 344 B1**
(45) Date of publication and mention of the grant of the patent: **18.11.1993**
(21) Application number: 87302864.1
(22) Date of filing: 02.04.1987
(51) Int. Cl.: A61K 39/395

(54) **Monoclonal antibodies and their use**
Monoklonale Antikörper und deren Verwendung
Anticorps monoclonaux et leur application

(30) Priority: 02.04.1986 GB 8608068
(43) Date of publication of application: 07.10.1987
(60) Divisional application: 92118716.7
(73) Proprietor: THE WELLCOME FOUNDATION LIMITED, London NW1 2BP (GB)
(72) Inventor: Cobbold, Stephen Paul, Tennis Court Road, Cambridge CB2 1QP (GB); Clark, Michael Ronald, Tennis Court Road, Cambridge CB2 1QP (GB); Benjamin, Richard John, Tennis Court Road, Cambridge CB2 1QP (GB); Waldmann, Herman, Tennis Court Road, Cambridge CB2 1QP (GB)
(74) Representative: Baker-Munton, Nicola Jane

(56) References cited:
- EP-A- 200 412
- EP-A- 0 018 794
- EP-A- 0 117 114
- EP-A- 0 235 805
- JOURNAL OF EXPERIMENTAL MEDICINE, vol. 162, September 1985, The Rockefeller University Press; G.E.RANGES et al., pp. 1105-1110
- NATURE, vol. 312, 06 December 1984; S.P.COBBOLD etal., pp. 548-551
- JOURNAL OF EXPERIMENTAL MEDICINE, vol. 162, July 1985, The Rockefeller University Press; R.L.KIRKMAN et al., pp. 358-362
- THE JOURNAL OF IMMUNOLOGY, vol. 135, no. 3, September 1985, The American Association of Immunologists, US; D.WOFSY et al., pp. 1698-1701
- THE JOURNAL OF IMMUNOLOGY, vol. 126, no. 4, April 1981, The Williams & Wilkins Co., US; T.UCHIYAMA et al., pp. 1393-1397
- THE JOURNAL OF IMMUNOLOGY, vol. 134, no. 2, February 1985, The American Association of Immunologists, US; D.WOFSY et al., pp. 852-856
- SCIENCE, vol. 227, 1985; M.K.WALDOR et al., pp. 415-417
- CHEMICAL ABSTRACTS, vol. 106, no. 3, 19 January 1987, Columbus, OH (US); J.D.SEDGWICK et al., no. 16880e
- THE NEW ENGLAND JOURNAL OF MEDICINE, vol. 305, 06 August 1981; A.BENEDICT COSIMI et al., pp. 308-314
- JOURNAL OF EXPERIMENTAL MEDICINE, vol. 164, 1986, The Rockefeller University Press, US; pp. 911-925

## Description

This invention relates to monoclonal antibodies and their use.

A major goal in immunology has been to find a means of selectively abolishing an individual's potential to mount an immune response to certain antigens, while preserving responsiveness to others. The facility to induce such specific immunological unresponsiveness in an adult would have major implications for tissue-grafting, the control of allergy and for treatment of autoimmune disease.

It has been shown that immunosuppresive regimes, such as irradiation, anti-lymphocyte globulin or thoracic duct drainage, may facilitate tolerance induction. Reference may be had in this context to papers by W.O. Wiegle, Adv. immun., 16, 61-121 (1973) and by G.R. Shellam, Immunology, 17, 267-270 (1969).

The preparation of rat monoclonal antibodies, designated YTS 191.1, directed to the L3T4 (CD4) molecule on T-helper cells of mice has been reported by S.P. Cobbold et al, Nature, 312, 548-551 (1984). Moreover D.P. Dialynas et al have described properties of rat monoclonal antibodies, designated GK 1.5, also directed to the L3T4 molecule on T-helper cells, in J. Immun., 131, 2445-2451 (1983), and in Immun. Rev. 47, 29-56 (1983). The L3T4 molecule is the name given to the mouse CD4 molecule. There is a CD4 type molecule on T-helper cells in all mammalian species. Treatment of mice with such monoclonal antibodies leads to rapid depletion of T-helper cells from the blood and lymphoid tissues, with subsequent immunosuppression of a range of immune functions. This latter work has been reported by S.P. Cobbold et al, loc cit, and by D.C. Wofsy et al, J. Immun., 135, 1698-1701 (1985). In this reported work the long term effects of such monoclonal antibodies were studied. D.C. Wofsy et al, loc cit, further reported that L3T4 depleted animals fail to respond on immunisation with protein antigens.

Ranges et al., J. Exp. Med., 162, 1105-1110 (1985) reported experiments in which they found that treatment with an anti-L3T4 monoclonal antibody significantly decreased incidence and delayed onset of type II collagen-induced arthritis in mice. This effect appears to result from generalised damage to the immune system (immunosuppression).

Waldor et al., Science, 227, 415-417 (1985) disclose that administration of an anti-L3T4 monoclonal antibody prevented development of allergic encephalomyelitis in mice. Again the effect appears to be a general immunosuppressive effect.

EP-A-0118794 (Ortho) relates to the production of a hybridoma secreting monoclonal antibodies against OKT4 antigen on normal peripheral helper T-cells. It is proposed, inter alia that the monoclonal antibody can be used to treat autoimmune disease caused by excess of helper T-cells. Again the effect is clearly an immunosuppressive effect caused by elimination of helper T-cells.

We have now discovered, surprisingly, that certain autoimmune diseases in mammals respond favourably to short term treatment with monoclonal antibodies to the CD4 molecule on helper T-cells.

In addition, we have surprisingly, discovered that certain classes of monoclonal antibodies can produce tolerance to a primary antigen. If mammals, such as mice, are exposed to primary antigen challenge during or shortly before a brief period of anti-CD4 therapy, then the treated subject develops tolerance towards that primary antigen after the anti-CD4 therapy has been completed when the challenge with the primary antigen is renewed.

The present invention provides the use of a monoclonal antibody directed against the CD4 antigen on T-helper lymphocytes for the manufacture of a medicament for the treatment of a mammal to induce tolerance in the said mammal to a primary antigen, the treatment comprising administering to the subject mammal sufficient of the medicament to deplete significantly the population of T-helper lymphocytes in the subject mammal, challenging the subject mammal with the primary antigen and allowing the population of T-helper lymphocytes in the subject mammal to re-establish itself in the presence of the primary antigen so that tolerance to the primary antigen is established.

The period between exposure to the antigen and commencement of the antibody treatment should not be such as to allow the mammal to develop a strong immune response and should preferably not exceed 5 days.

As examples of mammals there can be mentioned mice, cats, dogs, horses, cows and man.

The amount of anti-CD4 monoclonal antibodies administered should be sufficient for the mammal's helper T-cell population to be significantly depleted.

In the treatment of a subject mammal with anti-CD4 monoclonal antibody, treatment is typically carried out so as to reduce the population of helper T-lymphocytes to less than about 20% and preferably to less than about 10% of their normal level. In mice such reduction of the population of helper T-lymphocytes can be achieved, for example, by administration of the antibodies in a series of doses each of the order of about 20mg antibody per kg of body weight. Administration of the antibody can be effected by intravenous or intraperitoneal injection in one or more doses.

Sufficient of the antibody is administered in one or more doses to the subject mammal to cause its population of helper T-lymphocytes to be depleted for a limited period. Generally speaking this limited period should be at least 1 day but need not exceed about 14 days. It will normally suffice, so our experiments to date would appear to suggest, to administer the antibody for a period of from 1 to about 7 days, e.g. 3 days. Conveniently the doses of antibody are selected so as to obtain the desired depletion of helper T-lymphocytes by daily injections.

Whilst a specific population of T-lymphocytes is depleted in the subject mammal as a result of administration of the anti CD4 antibody, an antigen may be administered to the subject mammal. Such antigen may be administered by injection, by inhalation, by ingestion, by transplantation or by implantation. It may comprise, for example, an immunoglobulin.

In our experiments we have shown that mice injected with antigen under the umbrella of an anti-L3T4 (CD4) antibody maintain a record of the encounter manifesting as tolerance, and we have been able to induce tolerance in normal adult mice with an immunogenic form of an antigen.

In contrast to other rat IgG2b monoclonal antibodies that deplete lymphocyte populations, anti-L3T4 (CD4), such as YTS 191.1 and GK1.5, both of which are rat IgG2b monoclonal antibodies, do not elicit anti-globulin responses in vivo, according to S.P. Cobbold et al., Nature, 312, 548-551 (1984), D.C. Wofsy et al., J. Immun., 135, 1698-1701 (1985) and D. Wofsy et al., J. Exp. Med., 161, 378-391 (1985) as well as S.P. Cobbold et al., Adv. Exp. Med. Bio., 186, 789-795 (1985).

The tolerogenic properties of the anti-CD4 antibody have important implications for serotherapy with monoclonal antibodies. By treatment of patients with suitable anti-L3T4 (CD4) monoclonal antibodies at the beginning of such serotherapy it becomes possible to diminish substantially and in some cases to suppress entirely the anti-globulin response normally mounted by the patient's body to the monoclonal antibodies used in such serotherapy.

We have also demonstrated that anti-CD4 monoclonal antibodies have an important role to play in correction of the abnormal immune response associated with many forms of autoimmune disease. Although prior reported work has studied the immunosuppressive effects of anti-L3T4 monoclonals, as reported by D. Wofsy et al., J. Exp. Med., 161, 378-391 (1985), by M.K. Waldor et al., Science, 227, 415-417 (1985), by G.E. Ranges et al., J. Exp. Med., 162, 1105-1110 (1985), and by S.W. Brostoff et al., J. Immun., 133, 1938-1942 (1985), and has demonstrated the value of anti-L3T4 (CD4) monoclonals for amelioration of autoimmune diseases in mice and rats, it is surprising that a short burst of anti-L3T4 (CD4) therapy using anti-CD4 monoclonal antibodies can produce long term beneficial effects in treatment of autoimmune diseases. Thus we have shown that so-called "lupus mice", i.e. mice suffering from an autoimmune disease having many of the same symptoms as lupus erythematosus in human beings, have their symptoms ameliorated by injection at the age of four months with daily doses of two anti-L3T4 (CD4) monoclonal antibodies, YTS 191.1 and YTA 3.1 (equivalent to 0.4 mg of monoclonal antibody per dose) for 1 week. There were no deaths in the treated group within 6 months thereafter, although no special precautions were taken (e.g. administration of antibodies or sterilization of their environment), whereas at least 80% of the control animals had died by this time.

In another set of experiments we have shown that the immune response of mammals to a primary antigen can be modified by a short course of injections with anti-CD4 monoclonal antibodies begun shortly after the first challenge with the antigen. Thus mice given human γ-globulin as a primary antigen develop tolerance towards it when given subsequently, up to 5 days later, a short course of treatment with monoclonal antibodies to the L3T4 molecule on mice helper T-lymphocytes. Such a short course of treatment may comprise, for example, daily doses for from 3 to 7 days.

Allogenic reactions are the major limitation to organ transplants. These are manifested as rejection of the grafted tissue and also in the case of bone marrow transplantation, grant-versus-host disease. By using a combined therapy of CD4 monoclonals and CD8 monoclonals we have been able to produce long term tolerance to skin grafts differing in multiple minor transplantation antigens. Our experiments show that anti-L3T4 (CD4) therapy gave prolonged graft survival, but also demonstrated that optional tolerance induction by anti-L3T4 (CD4) will sometimes require the addition of other immunosuppressive agents.

A number of experiments have demonstrated that there are situations where it is insufficient to use monoclonal antibodies against L3T4 (CD4) alone to induce complete immunological tolerance. This is because the L3T4 (CD4) positive T-cells are solely responsible for helping the B-cells produce antibody, but other T-cells which are L3T4 (CD4) negative are separately involved in different types of immune responses. These other T-cells also express specific molecules on their cell surfaces which can be recognised by monoclonal antibodies, in particular, Lyt-2 (CD8) and the IL-2 receptor (CD25). We have shown, in a number of experiments in mouse models, that monoclonal antibodies to Lyt-2 or the IL2 receptor can be used to induce tolerance to protein antigens and even to mismatched tissue grafts when used in combination with the L3T4 (CD4) monoclonal antibodies.

The invention is further illustrated in the following Examples:

### Example 1

This Example demonstrates the return of immunocompetence after YTS 191.1 (anti-L3T4 (a CD4)) treatment in euthymic mice.

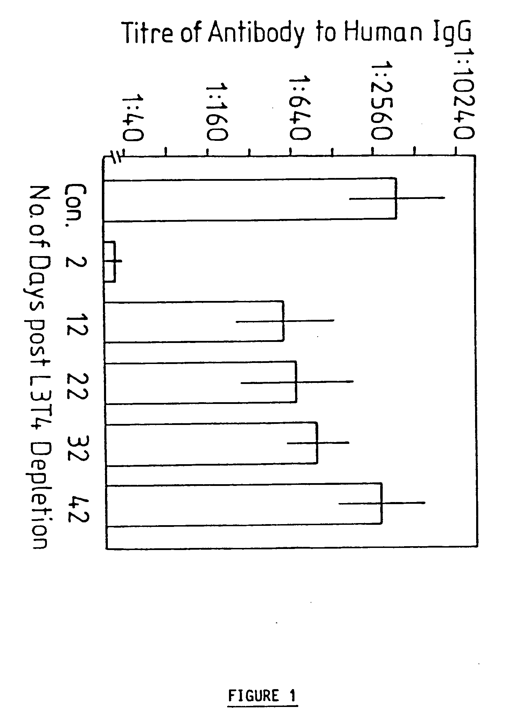

Male CBA/Ca mice aged 8-12 weeks were depleted of L3T4-positive T-cells by daily injections on days 0 (intravenous (iv.)), 1 and 2 (intraperitoneal (i.p.)) of YTS 191.1 as 0.2ml of ascitic fluid from (DA x LOU) F₁, rats enriched for immunoglobulin by precipitation with 50% saturated ammonium sulphate, and equivalent to 0.4mg of active monoclonal antibody per dose. Controls received saline by the same protocol. Groups of four mice were randomly selected on various days following treatment and immunized with 0.5mg heat-aggregated HGG i.v., purified from group 0 rhesus-positive serum by ammonium sulphate precipitation followed by elution through an ion-exchange chromatographic column (DE52, Whatman) in 0.01 sodium phosphate buffer pH 8.0, dialysed into phosphate-buffered saline (PBS) at 10mg mlR-1F, and then aggregated by heating to 63° for 25 minutes followed by overnight incubation on ice (see W.O. Weigle, Adv. Immun., 16, 61-121 (1973)). Mice were bled from their tail veins 11 days after immunization and the sera stored at -20°C. Serum anti-HGG antibody titres were measured by an enzyme-linked immunosorbent assay (ELISA) as follows. Polyvinyl microtitration trays were coated with purified HGG by incubation of 50µl per well of 20µg ml⁻¹ HGG in PBS for 60 minutes at 37°, washed three times in PBS/0.05% (v/v) Tween 20 (Sigma), and then blocked overnight with 1% (w/v) bovine serum albumin (BSA) in PBS/0.02% (w/v) sodium azide. The trays were then washed and doubling diluations of test sera in 0.1% BSA/PBS were added to each tray at 50µl per well and incubated in 60 minutes at 20°C. A positive control of peroxidase-linked rabbit anti-human immunoglobulin (Dako) was titrated on each tray. The test samples were followed, with intervening washes, by species-specific biotinylated sheep anti-mouse immunoglobulin (Amersham) and then biotinylated streptavidin horseradish peroxidase complex (Amersham) both at 1:1,000 dilution in PBS/0.1% BSA and incubated at 50µl per well for 35 minutes at 20°C. Trays were then washed and developed for 5 min with o-phenylenediamine (100 l per well) and the reaction stopped with 50µl per well of 0.5 M H₂SO₄. Absorbance was read at 490nm, the results plotted graphically and the titres read relative to the positive control. Data are geometric mean ±s.d. of the antibody titres from four mice. The results are summarised in Figure 1. They show that mice challenged with heat-aggregated human γ-globulin (HGG) 2 days after L3T4 depletion generated, as expected, no primary antibody response. However, challenge at day 12 elicited a strong anti-HGG response and at day 42 this response was equivalent to that in untreated controls.

### Example 2

This Example shows that YTS 191.1 (anti-L3T4 (a CD4)) induces unresponsiveness to its own IgG2b epitopes and to heat-aggregated HGG given concomitantly.

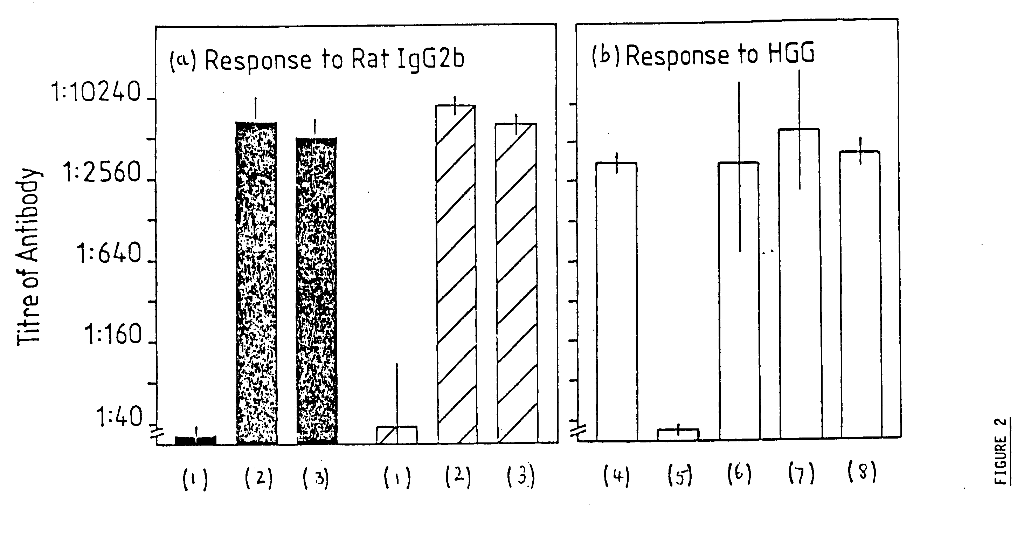

Adult CBA/Ca male mice in groups of five were pretreated with three injections of YTS 191.1 as in Example 1 (groups 1, 4, 5), or as controls received an irrelevant IgG2b monoclonal antibody (YTH 65.3.3, anti-human T200 obtained by the method of C. Bindon et al, Transplanation, 40, 538-543 (1985)) (groups 2,6) or saline (groups 3, 7, 8) by the same protocol. Groups 5, 6 and 7 were then injected i.p. on days 2 and 3 with 0.5mg doses of heat-aggregated HGG prepared as in Example 1. Following this pretreatment, mice were maintained on antibiotics (oxytetracyclin 50mgl⁻¹, Terramycin; Pfizer) and were free of any obvious disease. On day 12 all mice were bled and the effectiveness of anti-L3T4 treatment was confirmed by the absence of an antibody response to HGG in group 5 as compared with strong primary responses in group 6 and 7 measured by an ELISA. On days 42 and 52, mice were immunized i.p. with 0.5mg of the heat-aggregated form of YTH 3.2.6 (anti-CD7 IgG2b), of YTH 53.1.4 (anti-human red blood cell, IgG2b), or of HGG. YTH 3.2.6 was prepared by the method described by H. Waldmann et al, Adv. Exp. Med. Biol., 186, 869-875 (1985). YTH 3.2.6 and YTH 53.1.4 were purified from (DA x LOU0F₁ rat ascites by ammonium sulphate precipitation, followed by ion-exchange chromtography in 0.01M sodium phosphate buffer pH 8.0 (DE 52, Whatman) and then heat-aggregated as above. Mice were bled from their tail veins on day 58 and their sera stored at -20°C. The serum titres of antibody to the respective immunizing Ag (YTH 3.2.6, YTH 53.1.4 or HGG) were determined by ELISA as in Example 1. A mixture of mouse monoclonal antibodies against rat immunoglobulin (Norig 7.16.2, Norig 1.1.6, both anti-rat IgG2b obtained as described by N.M. Agel et al, J. Immun. Meth 69, 207-214 (1984)) and mar 18.5 (anti-rat light chain obtained as described by L.L. Lanier et al, Hybridoma, 1, 125-130 (1982)) was used as a reference positive control for measurement of the anti-rat IgG2b antibody responses. Data are geometric means ±s.d. of antibody titres from five mice. The results are plotted in Figures 2(a) and (b). As shown in Figure 2(a), when mice were pre-treated with YTS 191.1, or an irrelevant monoclonal antibody or saline, and then 42 days later were immunized with either of two different heat-aggregated IgG2b monoclonals, having no binding activity for mouse cells (YTH 3.2.6 and YTH 53.1.4), no anti-globulin responses could be detected in anti-L3T4 - treated mice (group 1) compared with controls (groups 2 and 3). Similarly, administration of HGG under the anti-L3T4 umbrella led to later unresponsiveness to re-challenge with this antigen (group 5 in Figures 2(b)). Mice receiving anti-L3T4 but no initial "recording" dose of HGG (group 2), responded as well as controls (groups 6 to 8) to the late HGG challenge.

Similar results were obtained with HGG derived from a different donor. Later bleeds did not expose immune responses in tolerant mice.

### Example 3

This Example demonstrates the specificity of unresponsiveness to rat IgG2b and HGG.

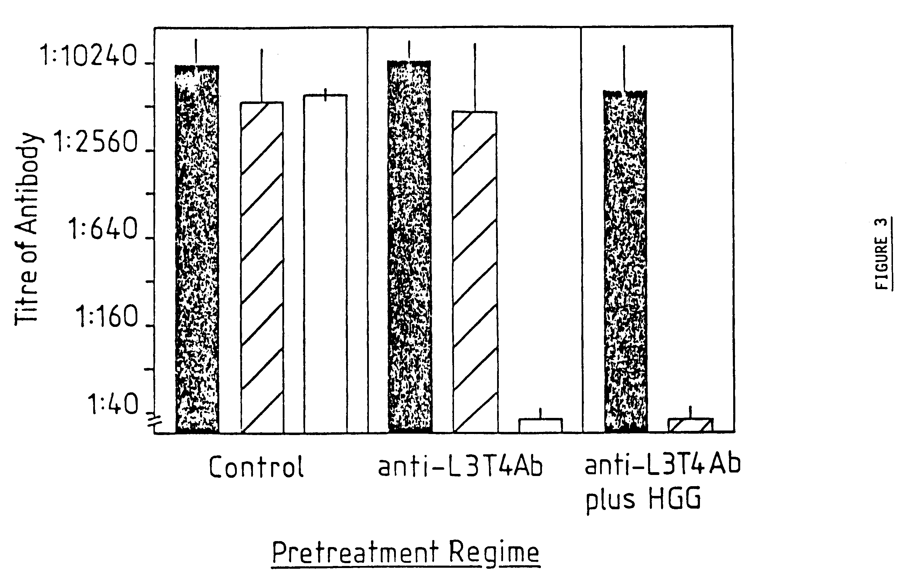

Adult male CBA/Ca mice were rendered unresponsive to rat IgG2b by YTS 191.1 (anti-L3T4 antibody) pretreatment as in group 1 in Figure 2(a) or to HGG by pretreatment with YTS 191.1 plus aggregated HGG (anti-L3T4 antibody plus HGG) as in group 5 in Figure 2(b). On days 42 and 52 after induction of unresponsiveness, these mice are age-matched controls were immunized with 0.5mg i.p. of the heat-aggregated form of HGG, CGG or YTH 3.2.6 (rat IgG2b), and then bled on day 58. Chicken γ-globulin was obtained from Miles Laboratories and heat-aggregated as above. Sera were stored at -20°C and antibody titres determined by ELISA. Data are geometric means ±s.d. of antibody titres from five mice. The results were plotted in Figure 3. They show that mice rendered unresponsive to rat Igg2b by administration of anti-L3T4 remained fully responsive to HGG or chicken γ-globulin (CGG). Similarly, mice made unresponsive to HGG given CGG under the anti-L3T4 unbrella responded normally to CGG. Hence the state of unresponsiveness appears to be true tolerance to the administered antigens.

### Example 4

This example demonstrates the combined use of L3T4 (CD4), Lyt-2 (CD8) and IL-2 (CD25) receptor monoclonal antibodies to generate tolerance to mismatched skin grafts.

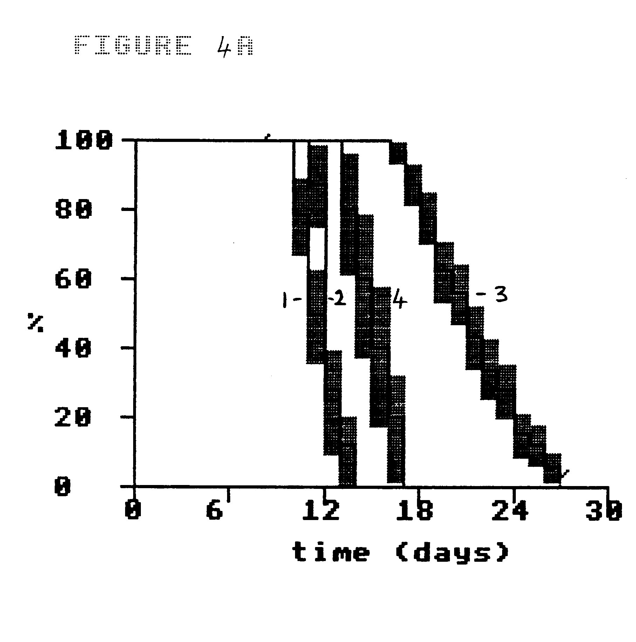

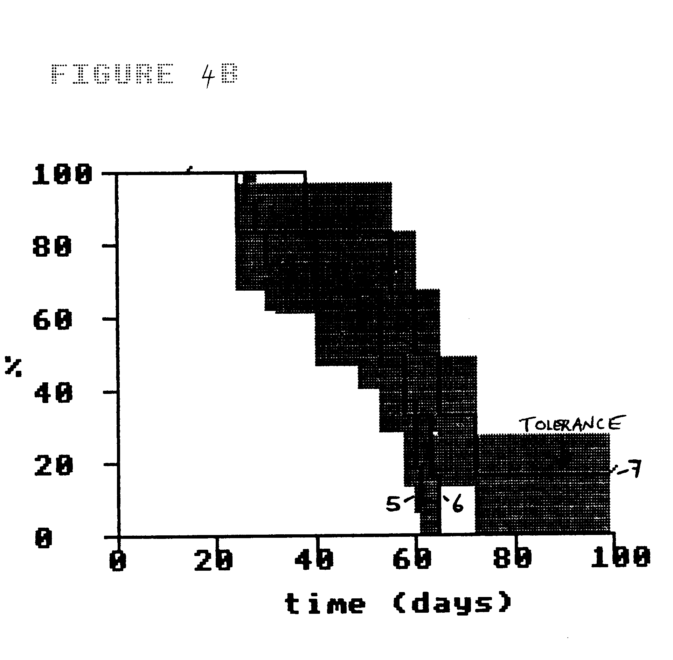

Male CBA/Ca mice aged 8-12 weeks were given tail skin grafts from mismatched BALB/c mice, together with monoclonal antibody injections three times per week for four weeks. The first five injections were given intravenously, while subsequent injections were intraperitoneally. Figure 4a shows the following groups: control group 1 received no antibody; group 2 received Lyt-2 (CD8) antibodies (TYS 169.4.2 + YTS 156.7.7, 25µg each per injection); group 3 received L3T4 (CD4) antibodies (YTS 191.1.2 + YTA 3.1, 25µg each per injection); group 4 received anti-IL-2 receptor antibody (YCTLD 45.1, 200µg per injection). It can be seen that none of the antibodies alone was sufficient to generate tolerance to the skin grafts, although anti-L3T4 gave a significant prolongation in graft survival. Figure 4b shows that adding the anti-L3T4 and anti-Lyt-2 (group 5) or anti-L3T4 anti-IL-2 receptor antibodies (group 6) together (in the same doses as above) gave a much improved graft survival. Only the combination of all three types of monoclonal antibody allowed any of the mice to maintain the mismatched skin grafts indefinitely and without further treatment (group 7). This demonstrates that optimal tolerance induction by anti-L3T4 (CD4 antibodies) will sometimes require the addition of further immunosuppressive agents, in particular other monoclonal antibodies.

### Example 5

The following Example shows a typical pharmaceutical preparation for preparing a tolerogenic vaccine using tolerogenic monoclonal antibodies.

The tolerogenic vaccine is sterile, pyrogen-free, and free from other abnormal toxicity and will be presented in a lyopholised form in sterile vials.

Each vial contains approximately 5mg of sterile freeze-dried tolerogenic monoclonal. The exact amount of monoclonal will be indicated on each vial.

The antibody is freeze-dried from 2ml of a solution containing sodium chloride (0.2M), L-histidine (20mM), sorbitol (1%w/v), and human serum albumin (1%w/v). Therefore, each vial contains 23.4mg sodium chloride, 6.2mg L-histidine, and 20mg each of sorbitol and human serum albumin, in addition to the monoclonal.

The vials should be stored in a fridge at 2-8°C until reconstitution.

They should be used as soon as possible after reconstitution in 2ml of water for injections.

### Example 6

The following exemplifies a combined pharmaceutical formulation of the present invention comprising a tolerogenic monoclonal and a therapeutic monoclonal antibody.

The formulation is sterile, pyrogen free, and free from abnormal toxicity and is presented in a lyopholised form in sterile vials.

The tolerogenic antibodies (e.g. anti-CD4) and therapeutic antibody (e.g. anti-CD8) will be dissolved in a solution containing sodium chloride (0.2 M), L-histidine (20 mM), sorbitol (1% w/v), and human serum albumin (1% w/v). The solution will then be subjected to freeze-drying.

## Claims

1. The use of a monoclonal antibody directed against the CD4 antigen on T-helper lymphocytes for the manufacture of a medicament for the treatment of a mammal to induce tolerance in the said mammal to a primary antigen, the treatment comprising administering to the subject mammal sufficient of the medicament to deplete significantly the population of T-helper lymphocytes in the subject mammal, challenging the subject mammal with the primary antigen and allowing the population of T-helper lymphocytes in the subject mammal to re-establish itself in the presence of the primary antigen so that tolerance to the primary antigen is established.

2. The use according to Claim 1 wherein the primary antigen is an autoantigen or a transplanted antigen.

3. The use according to Claim 1 or 2 wherein the mammal is man.

4. The use according to any of Claims 1 to 3 wherein the treatment also includes administration of another immunosuppressive agent.

5. The use according to Claim 4 wherein the other immunosuppressive agent is an anti-CD8 and/or anti-CD25 monoclonal antibody.

## Patentansprüche

1. Verwendung eines monoklonalen Antikörpers, der gegen das CD4-Antigen auf T-Helfer-Lymphocyten gerichtet ist, für die Herstellung eines Medikaments für die Behandlung eines Säugers zur Induzierung von Toleranz in dem Säuger gegenüber einem primären Antigen, wobei die Behandlung umfasst: Verabreichen einer ausreichenden Menge des Medikaments an den Säuger, wodurch die Population der T-Helfer-Lymphocyten in dem Säuger signifikant verringert wird, Exposition des Säugers mit dem primären Antigen und Ermöglichen, dass die Population der T-Helfer-Lymphocyten in dem Säuger sich selbst in Anwesenheit des primären Antigens wieder bildet, so dass Toleranz gegenüber dem primären Antigen begründet wird.

2. Verwendung nach Anspruch 1, wobei das primäre Antigen ein Autoantigen oder ein transplantiertes Antigen ist.

3. Verwendung nach Anspruch 1 oder 2, wobei der Säuger der Mensch ist.

4. Verwendung nach einem der Ansprüche 1 bis 3, wobei die Behandlung auch Verabreichung eines anderen Immununterdrückungsagenses umfasst.

5. Verwendung nach Anspruch 4, wobei das andere Immununterdrückungsagens ein Anti-CD8- und/oder Anti-CD25-monoklonaler Antikörper ist.

## Revendications

1. Utilisation d'un anticorps monoclonal dirigé contre l'antigène CD4 sur les lymphocytes T auxiliaires pour la fabrication d'un médicament destiné au traitement d'un mammifère pour induire la tolérance chez ce mammifère à l'égard d'un antigène primaire, le traitement comprenant l'administration à ce mammifère d'une quantité suffisante de ce médicament pour épuiser sensiblement la population des lymphocytes T auxiliaires chez ce mammifère, l'attaque du mammifère avec l'antigène primaire et l'admission de la population des lymphocytes T auxiliaires du mammifère à se rétablir en présence de l'antigène primaire de façon à établir la tolérance à l'égard de l'antigène primaire.

2. Utilisation suivant la revendication 1, dans laquelle l'antigène primaire est un autoantigène ou un antigène transplanté.

3. Utilisation suivant la revendication 1 ou 2, dans laquelle le mammifère est un être humain.

4. Utilisation suivant l'une quelconque des revendications 1 à 3, dans laquelle le traitement inclut aussi l'administration d'un autre agent immunosuppresseur.

5. Utilisation suivant la revendication 4, dans laquelle l'autre agent immunosuppresseur est un anticorps monoclonal anti-CD8 et/ou anti-CD25.
